# EUROPEAN PATENT SPECIFICATION

(11) **EP 3 324 817 B1**
(45) Date of publication and mention of the grant of the patent: **30.12.2020**
(21) Application number: 15790479.8
(22) Date of filing: 16.10.2015
(51) Int. Cl.: G16H 30/40

(54) **OPHTHALMIC SURGICAL IMAGE PROCESSING**
OPHTHALMISCHE CHIRURGISCHE BILDVERARBEITUNG
TRAITEMENT D'IMAGES OPHTALMIQUES CHIRURGICALES

(43) Date of publication of application: 30.05.2018
(73) Proprietor: Alcon Inc., 1701 Fribourg (CH)
(72) Inventor: ABT, Niels A., 8203 Schaffhausen (CH)
(74) Representative: Hanna Moore + Curley
(86) International application number: PCT/EP2015/074032
(87) International publication number: WO 2017/063714

(56) References cited:
- EP-A1- 2 645 701
- EP-A1- 2 806 402
- WO-A1-2014/074595
- US-A1- 2012 098 926

## Description

### TECHNICAL FIELD

The present disclosure relates to ophthalmic surgery, and more specifically, to systems for and methods of processing an image received from a system containing at least one lens that inverts a portion of the image.

### BACKGROUND

Ophthalmic surgery saves and improves the vision of tens of thousands of patients every year. However, given the sensitivity of vision to even small changes in the eye and the minute and delicate nature of many eye structures, ophthalmic surgery is difficult to perform and the reduction of even minor or uncommon surgical errors or modest improvements in accuracy of surgical techniques can make an enormous difference in the patient's vision after the surgery.

One type of ophthalmic surgery, vitreoretinal surgery, encompasses various delicate procedures involving internal portions of the eye, such as the vitreous humor and the retina. Different vitreoretinal surgical procedures are used, sometimes with lasers, to improve visual sensory performance in the treatment of many eye diseases, including epimacular membranes, diabetic retinopathy, vitreous hemorrhage, macular hole, detached retina, and complications of cataract surgery, among others.

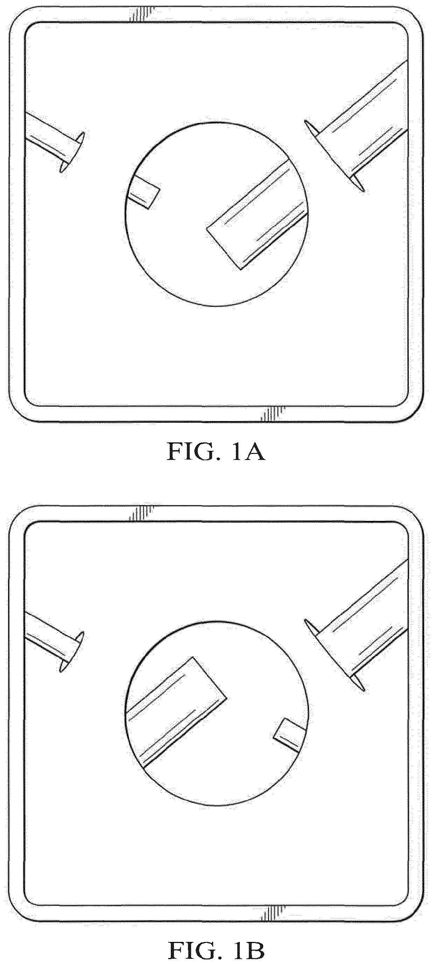

During vitreoretinal surgery, an ophthalmologist typically uses a surgical microscope to view the fundus through the cornea, while surgical instruments that penetrate the sclera may be introduced into the surgical field to perform any of a variety of different procedures (FIG. 1A). The surgical microscope provides imaging and optionally illumination of the fundus during vitreoretinal surgery. The patient typically lies supine under the surgical microscope during vitreoretinal surgery and a speculum is used to keep the eye exposed. Depending on a type of optical system used, the ophthalmologist has a given field of view of the fundus, which may vary from a narrow field of view to a wide field of view that can extend to peripheral regions of the fundus. For many types of vitreoretinal surgery using the surgical microscope, the surgeon may desire to have a very wide field of view of the fundus that extends beyond the equator and even out to the ora serrata.

Many such systems use a primary lens, such as an indirect lens, that inverts a portion of the image of the surgical field seen by the surgeon. Absent further correction, this type of system provides the surgeon an inverted view of the ends of the instruments in the surgical field (FIG. 1B). The surgeon must mentally correct the view in order to move the instruments properly. Such mental corrections are difficult to perform and negatively impact surgical outcomes in many instances. To render the situation even more difficult, most systems also provide the surgeon with a view of an area of the eye outside of the indirect lens field. In this area, the bodies of the surgical instruments are oriented correctly (FIG. 1B).

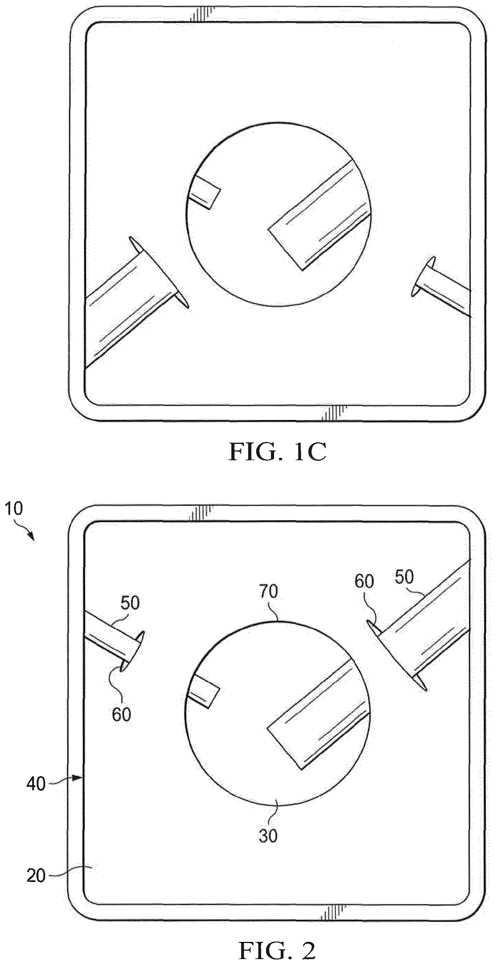

To at least render the ends of the surgical instruments as they are actually positioned, many systems, such as stereo digital inverters, contain an inverter lens or perform digital image processing that further inverts the image of the surgical field. This results in the ends of the surgical instruments being portrayed accurately within the surgical field, but portions of the image that were not previously inverted then become inverted, again resulting in a disconnect between the bodies of the surgical instruments in part of the surgical field, and the ends of the instruments. The resulting image (FIG. 1C), as compared to the actual location of the instruments (FIG. 1A), can be seen by comparing FIG. 1A and FIG. 1C. Although this type of image requires less mental correction by the surgeon, the inverted portrayal of part of the bodies of the instruments is distracting and requires mental correction. As a result, surgical outcomes are still negatively impacted in many instances.

### SUMMARY

It will be appreciated that the scope is in accordance with the claims. Accordingly, there is provided a system for performing ophthalmic surgery, as defined in claim 1. Further features are provided in accordance with the dependent claims. The specification may include description of arrangements outside the scope of the claims provided as background and to assist in understanding the invention.

The present specification provides a system for performing ophthalmic surgery. The system includes a primary lens that inverts a portion of an image of a surgical field through which light reflected from an eye undergoing ophthalmic surgery passes. The system also includes an image sensor that converts the light passed through the primary lens into a lens image and light that has not passed through the primary lens into a peripheral image. The lens image and the peripheral image form a digital image. The system further includes a processing resource that identifies a boundary between the lens image and the peripheral image in the digital image and inverts at least a portion of the lens image or at least a portion of the peripheral image to form a corrected image. In addition, the system includes a display that displays the corrected image.

In additional arrangements of the specification, which may be combined with the system above and with one another unless clearly mutually exclusive, no portion of the corrected image is inverted as compared to the eye or less than 10% of the corrected image is inverted as compared to the eye. There may be at least one surgical instrument in the eye undergoing ophthalmic surgery and no portion of the corrected image containing a surgical instrument is inverted as compared to the surgical instrument or less than 10% of the corrected image is inverted as compared to the surgical instrument. The lens may contain an identifier that is detected by the processing resource to identify the boundary. The processing resource may use huge circle detection to identify the boundary. The processing resource may use continuity detection to identify the boundary.

The specification further provides a method of performing ophthalmic surgery by placing a primary lens between the eye and an image sensor such that the image sensor receives light reflected from the eye that passes through the lens and light reflected from the eye that does not pass through the lens, converting the light received by the image sensor to a digital image containing a lens image converted from light that passes through the lens and a peripheral image converted from light that does not pass through the lens, identifying a boundary between the lens image and the peripheral image using a processing resource, inverting at least a portion of the lens image or at least a portion of the peripheral image to form a corrected image, and displaying the corrected image.

In additional arrangements of the specification, which may be combined with the system above and with one another unless clearly mutually exclusive no portion of the corrected image is inverted as compared to the eye, or less than 10% of the corrected image is inverted as compared to the eye. At least one surgical instrument may be inserted into the eye and no portion of the corrected image containing a surgical instrument is inverted as compared to the surgical instrument, or less than 10% of the corrected image is inverted as compared to the surgical instrument. The primary lens may contain an identifier and the method may include detecting the identifier and identifying the boundary using the identifier. The identifier may be located around the periphery of the lens, such that it corresponds with the boundary. The processing resource may use the identifier to obtain information regarding the dimensions of the lens from a database and may use the information regarding the dimensions to identify the boundary. Identifying the boundary may include the processing resource using huge circle detection. A dye may be introduced into the eye and color may be used in huge circle detection to identify the boundary and exclude non-lens circular or elliptical features. Identifying the boundary may include the processing resource using continuity detection. The processing resource may use two or more of an identifier located on the primary lens, huge circle detection, or continuity detection to identify the boundary.

The above systems and methods may further be used in combination with one another and with additional features described herein.

### BRIEF DESCRIPTION OF THE DRAWINGS

For a more complete understanding of the present invention and its features and advantages, reference is now made to the following description, taken in conjunction with the accompanying drawings, in which:
FIG. 1A is an image of the surgical field during an ophthalmic surgery, as known in the prior art;
FIG. 1B is an image portraying the surgical field during an ophthalmic surgery in which a portion of the image that passes through a primary lens is inverted;
FIG. 1C is an image portraying the surgical field during an ophthalmic surgery in which a portion of the image that does not pass through a primary lens is inverted;
FIG. 2 is a digitally processed image portraying the surgical field during an ophthalmic surgery in which the image contains components that pass through a primary lens and portions that do not, but no portion of the image is inverted;
FIG. 3 is a system for acquiring, digitally processing, and displaying an image of the surgical field in an ophthalmic surgery in which no portion is inverted;
FIG. 4A is a flow-chart of a method for receiving and digitally processing an image of the surgical field in an ophthalmic surgery to detect and correct inversions; and
FIG. 4B is a flow-chart of another method for receiving and digitally processing an image of the surgical field in an ophthalmic surgery to detect and correct inversions.

### DETAILED DESCRIPTION

The present disclosure relates to ophthalmic surgery, and more specifically, to systems for and methods of processing an image received from a system containing at least one lens that inverts a portion of the image to detect inverted portions of the image and reinvert only these portions to produce a final image without any inversions. Such an image more accurately portrays the surgical field, does not require mental correction of the surgical instrument positions by the surgeon, and decreases the difficulty of ophthalmic surgery, which may improve surgical outcomes for patients.

Referring now to the drawings, FIG. 2 presents a corrected image 10 of the surgical field during an ophthalmic surgery on eye 20. The image contains a lens image 30, which has passed through and been inverted by a primary lens, as well as a peripheral image 40, which has not passed through a primary lens. Surgical instruments 50 passing into eye 20 at insertion points 60 are visible in corrected image 10. Due to differences in magnification of lens image 30 and peripheral image 40, a lens boundary 70 may also be visible. FIG. 2 depicts a surgical field as shown in FIG. 1A. In FIG. 2, no portion of corrected image 10 is inverted. Alternatively, there may be no portion of corrected image 10 containing an instrument 50 is inverted. Further alternatively, less than 10% of corrected image 10 may be inverted; or less than 10% of the total portion of corrected image 10 containing an instrument 50 may be inverted. Although an image with no inversion may be easier to produce or better suited to reducing surgical errors, an image containing small inversions, particularly if these inversions do not include the surgical instruments or other visually distinguishable features may also be acceptable. This is especially true if these inversions do not require mental correction by the surgeon in order to perform the surgery or if tolerating these inversions provides advantages in quicker image processing, more accurate processing of portions of the image with distinguishable features, or image processing using simpler, more reliable, or cheaper equipment. For instance, portions of the image near lens boundary 70 may remain inverted without harm if they are too blurry or dark to provide useful information to the surgeon.

All or a part of corrected image 10 may be magnified. Typically lens image 30 is magnified between 5x and 40x, more particularly between 5x and 15x. Only lens image 30 may have been passed through a lens, all of corrected image 10 may have been passed through a lens, or portions of image 10 may have been passed through one or more lenses.

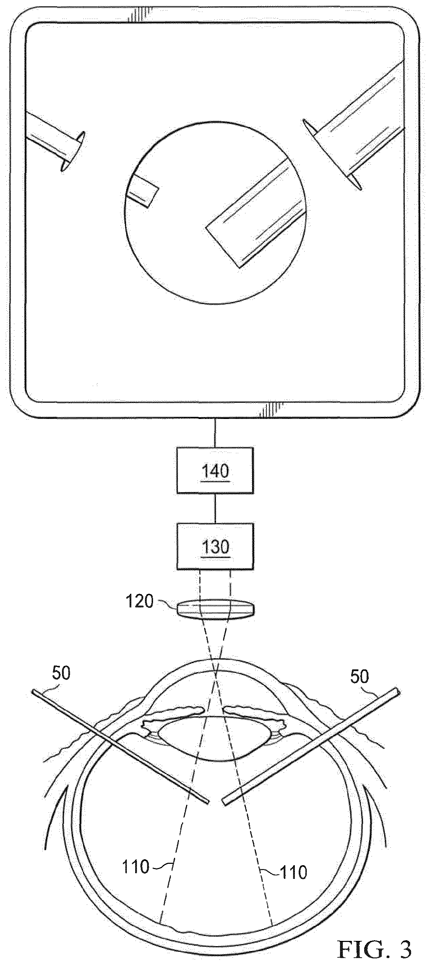

FIG. 3 presents an ophthalmic surgery system 100 for performing surgery on an eye 20 using surgical instruments 50. Reflected light beams 110 (shown as two representative beams only) pass out of eye 20 through primary lens 120 and are inverted by lens 120 in the process. Image sensor 130 intercepts light beams 110 as well as other light that has not passed through lens 130. Light beams 110 that have passed through lens 130 form lens image 30, while light that has not passed through lens 130 forms peripheral image 40. Image sensor 130 forms a digital image using this light to portray the surgical field of eye 20. Processing resource 140 detects the boundary of lens 120 and inverts the portion of the image found within this boundary. Finally, display 150 displays corrected image 10.

Although FIG. 3 depicts a system with only one lens 120 because such a system may be more reliable and cheaper to manufacture and maintain, systems with more than one lens are also possible. For instance, a system may contain a primary lens 120 and a second, inverter lens that inverts all of light before it reaches image sensor 130. In such a system, after the digital image is formed and the boundary of lens 120 is detected, the portion of the image found outside of the boundary is inverted.

Instruments 50 include any ophthalmic surgical instruments, particularly those used in vitreoretinal surgeries. Such instruments include cannulas, light probes, vitrectomy probes, forceps, scissors, laser probes, pics, and spatulas. Primary lens 120 may include any magnifying lens suitable for use in ophthalmic surgery, including direct and indirect contact lenses. System 100 may further contain instruments to position and focus lens 120. System 100 may also contain additional lenses to be used in conjunction with primary lens 120, or in place of primary lens 120. For instance, system 100 may include a wide-view lens that may be switched with primary lens 120. Lenses may be direct contact lenses or indirect lenses, as needed.

Image sensor 130 may be any electronic device able to convert light to a digital image. For instance, it may be a digital camera, a light-to-digital sensor, a semiconductor charge-coupled device (CCD), a complementary metal-oxide-semiconductor (CMOS) device, an N-type metal-oxide-semiconductor (NMOS) device, or another electronic device containing an array of photodiodes as part of one or more integrated circuits. Image sensor 140 may contain additional lenses or other elements to assist with image capture. Image sensor 130 produces a digital image with sufficient resolution to produce a usable corrected image 10, even after image processing.

Processing resource 140 may include any physical device able to store and run algorithms as described herein in order to produce corrected image 10. Although processing resource 140 is depicted separately form image sensor 130 in FIG. 3, they may be part of a single physical device, such as a single computer or set of integrated circuits.

Display 150 may include any type of screen or projector able to display corrected image 10 with sufficient resolution to be usable in an ophthalmic surgery. For instance, it may include any type of screen or projector used in connection with ophthalmic surgery, including displays of the type used in conventional vitreoretinal surgical systems that present digital images. In most instances, a special type of display for corrected image 10 as compared to other digital images used in ophthalmic surgery is not needed. Display 150 may display a single image as shown, or two images for stereoscopic viewing. Although display 150 is depicted separately from processing resource 140 and image sensor 130, it may be part of a single physical device, such as a single computer or set of integrated circuits, with processing resource 140, image sensor 130, or both.

System 100 may further contain other elements to facilitate its uses, such as memory to store corrected image 10 or instructions for processing resource 140, electrical connections, and hardware to position and focus any lenses, such as lens 120, and to position image sensor 130.

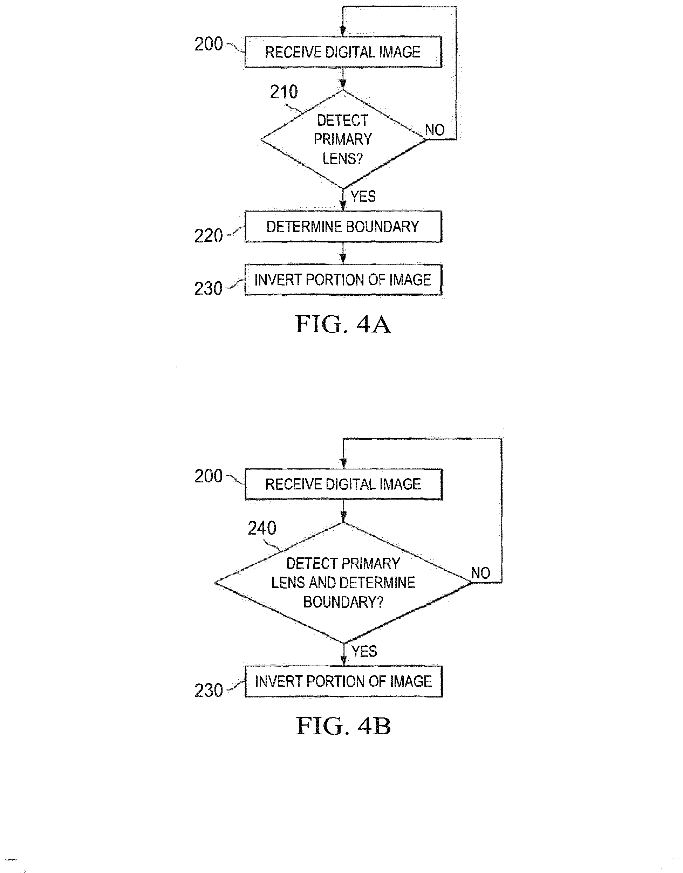

FIGs. 4A and 4B present a flow charts of a method for receiving and digitally processing an image according to the disclosure. In step 200, a digital image, such as that produced by image sensor 130 in system 100, is received by a processing resource. The digital image contains digital information representing light reflected from the surgical field in an ophthalmic surgery performed using surgical instruments, such as instruments 50. The image contains a lens image that has passed through a primary lens, such as primary lens 120, that inverts light beams passing through it to produce an inverted image. The image also contains a peripheral image that has not passed through a primary lens. One of the lens image and peripheral image is inverted and the other is not.

In FIG. 4A, in step 210, the processing resource detects the primary lens using a set of instructions stored in the processing resource or in memory in communication with the processing resource. If a primary lens is detected, then, in step 220, the processing resource determines the boundary of the primary lens within the image. In an alternative method shown in FIG. 4B, in step 240, boundary determination occurs concurrently with detection.

Finally, in step 230, a portion of the image is inverted to create a corrected image, such as corrected image 10. For instance, all of the lens image may be inverted or all of the periphery may be inverted. Alternatively, if instruments or other distinguishable features are also detected, only portions of the image containing these features may be inverted. The correct portion of the image to invert may be pre-set or, for methods used with highly variable systems that may sometimes contain only a primary lens and sometimes also contain an inverter lens, it may be input or determined based upon an identification of the lens or lenses present.

An image may be received in step 200 continuously or at set intervals.

This method and an associated methods to generate the image received in step 200 and display the image created in step 230 may take place in real-time or otherwise with a lag short enough to not hamper the ophthalmic surgery. For instance, the time between light reaching an image sensor, such as image sensor 130, and a corrected image corresponding to that light being displayed, such as on display 150, may be 100 milliseconds or less, 10 milliseconds or less, 4 milliseconds or less, or 1 millisecond or less.

In one method, the processing resource detects an identifier located on or on the lens, typically near its periphery. Typically the identifier is part of the image. The identifier may include a pattern, such as a bar code, not normally found in the eye. The identifier may be placed around the boundary of the primary lens, either continuously or at intervals, allowing the entire boundary to simply be detected by detecting the pattern.

Alternatively, the identifier may be placed in one location or not around the boundary and may identify the type of lens and its dimensions. It may further identify the dimensions and their locations with respect to the identifier. Processing resource 140 may then obtain information about the identified lens from a database and use that information to determine where the boundary is located in the image.

In another method, processing resource 140 may use huge circle detection, for example using the Hough transform, to locate the boundary, which will be substantially circular or elliptical. The set of instructions for huge circle detection may use additional information to exclude other, non-lens circular or elliptical features, such as bubbles. This additional information may include information regarding the type of lens used in the image and its dimensions. The additional information may also include color, which may differ between the exterior and interior of non-lens features, but not for the lens image and the peripheral image. For instance, any bubble-forming liquids in the eye may be dyed to facilitate this method.

In another method, processing resource 140 may use continuity detection to locate the boundary. Continuity detection may locate where features with regular edges, such as instruments, become discontinuous, which is often at the boundary. Continuity detection may be enhanced by using multiple images or continuously received images to detect movement as well. In such methods, changing angles of regular edges may be used to help locate features and places where features become discontinuous.

Any of the above methods used by processing resource 140 may be combined in step 220, in step 230, or in step 240. For instance, once a pattern has been used to identify a lens, huge circle detection may be used to help locate or verify the location of the boundary. In another example, the results of huge circle detection and continuity detection may be combined or used to verify one another.

## Claims

1. A system (100) for performing ophthalmic surgery, the system comprising:
a primary lens (120) that inverts a portion of an image of a surgical field through which light reflected from an eye undergoing ophthalmic surgery passes; the system being **characterised by**
an image sensor (130) that converts the light passed through the primary lens (120) into a lens image (30) and light that has not passed through the primary lens into a peripheral image (40), wherein the lens image (30) and peripheral image (40) form a digital image;
a processing resource that identifies a boundary between the lens image and the peripheral image in the digital image and inverts at least a portion of the lens image or at least a portion of the peripheral image to form a corrected image (10); and
a display (150) that displays the corrected image (10).

2. The system of claim 1, wherein no portion of the corrected image (10) is inverted as compared to the eye.

3. The system of claim 1, wherein less than 10% of the corrected image (10) is inverted as compared to the eye.

4. The system of any of claims 1 to 3, wherein there is at least one surgical instrument (50) in the eye undergoing ophthalmic surgery and no portion of the corrected image (10) containing a surgical instrument is inverted as compared to the surgical instrument.

5. The system of any one of claims 1 to 3, wherein there is at least one surgical instrument (10) in the eye undergoing ophthalmic surgery and less than 10% of the corrected image is inverted as compared to the surgical instrument.

6. The system of any preceding claim, wherein the lens contains an identifier that is detected by the processing resource (140) to identify the boundary.

7. The system of any preceding claim, wherein the processing resource (140) is configured to use huge circle detection to identify the boundary.

8. The system of any preceding claim, wherein the processing resource (140) is configured to use continuity detection to identify the boundary.

## Patentansprüche

1. System (100) zum Durchführen ophthalmischer Chirurgie, das System umfassend:
eine primäre Linse (120), die einen Abschnitt eines Bilds eines chirurgischen Felds, durch das von einem Auge, das einer ophthalmischen Chirurgie unterzogen wird, reflektiertes Licht verläuft, umkehrt;
das System **gekennzeichnet durch**
einen Bildsensor (130), der das durch die primäre Linse (120) verlaufene Licht in ein Linsenbild (30) und Licht, das nicht durch die primäre Linse verlaufen ist, in ein peripheres Bild (40) umwandelt, wobei das Linsenbild (30) und das periphere Bild (40) ein digitales Bild bilden;
ein Verarbeitungsbetriebsmittel, das eine Begrenzung zwischen dem Linsenbild und dem peripheren Bild in dem digitalen Bild identifiziert und mindestens einen Abschnitt des Linsenbilds oder mindestens einen Abschnitt des peripheren Bilds umkehrt, um ein korrigiertes Bild (10) zu bilden; und
eine Anzeige (150), die das korrigierte Bild (10) anzeigt.

2. System nach Anspruch 1, wobei kein Abschnitt des korrigierten Bilds (10) im Vergleich mit dem Auge umgekehrt wird.

3. System nach Anspruch 1, wobei weniger als 10 % des korrigierten Bilds (10) im Vergleich mit dem Auge umgekehrt werden.

4. System nach einem der Ansprüche 1 bis 3, wobei mindestens ein chirurgisches Instrument (50) in dem Auge, das einer ophthalmischen Chirurgie unterzogen wird, ist und kein Abschnitt des korrigierten Bilds (10), das ein chirurgisches Instrument enthält, im Vergleich mit dem chirurgischen Instrument umgekehrt wird.

5. System nach einem der Ansprüche 1 bis 3, wobei mindestens ein chirurgisches Instrument (10) in dem Auge, das einer ophthalmischen Chirurgie unterzogen wird, ist und weniger als 10 % des korrigierten Bilds im Vergleich mit dem chirurgischen Instrument umgekehrt werden.

6. System nach einem der vorhergehenden Ansprüche, wobei die Linse ein Kennzeichen enthält, das durch das Verarbeitungsbetriebsmittel (140) detektiert wird, um die Begrenzung zu identifizieren.

7. System nach einem der vorhergehenden Ansprüche, wobei das Verarbeitungsbetriebsmittel (140) konfiguriert ist zum Verwenden von Detektion großer Kreise, um die Begrenzung zu identifizieren.

8. System nach einem der vorhergehenden Ansprüche, wobei das Verarbeitungsbetriebsmittel (140) konfiguriert ist zum Verwenden von Kontinuitätsdetektion, um die Begrenzung zu identifizieren.

## Revendications

1. Système (100) pour effectuer des opérations de chirurgie ophtalmique, le système comprenant :
une lentille primaire (120) qui inverse une partie d'une image d'un champ chirurgical à travers lequel passe la lumière réfléchie par un œil subissant une chirurgie ophtalmique ; le système étant **caractérisé par** :
un capteur d'image (130) qui convertit la lumière passée à travers la lentille primaire (120) en une image de lentille (30) et la lumière qui n'a pas traversé la lentille primaire en une image périphérique (40), l'image de lentille (30) et l'image périphérique (40) formant une image numérique ;
une ressource de traitement qui identifie une limite entre l'image de lentille et l'image périphérique dans l'image numérique et qui inverse au moins une partie de l'image de lentille ou au moins une partie de l'image périphérique pour former une image corrigée (10) ; et
un dispositif d'affichage (150) qui affiche l'image corrigée (10).

2. Système selon la revendication 1, aucune partie de l'image corrigée (10) n'étant inversée par rapport à l'œil.

3. Système selon la revendication 1, moins de 10 % de l'image corrigée (10) étant inversée par rapport à l'œil.

4. Système selon l'une quelconque des revendications 1 à 3, au moins un instrument chirurgical (50) étant présent dans l'œil subissant une chirurgie ophtalmique et aucune partie de l'image corrigée (10) contenant un instrument chirurgical n'étant inversée par rapport à l'instrument chirurgical.

5. Système selon l'une quelconque des revendications 1 à 3, au moins un instrument chirurgical (10) étant présent dans l'œil subissant une chirurgie ophtalmique et moins de 10 % de l'image corrigée étant inversée par rapport à l'instrument chirurgical.

6. Système selon l'une quelconque des revendications précédentes, la lentille contenant un identifiant qui est détecté par la ressource de traitement (140) pour identifier la limite.

7. Système selon n'importe quelle revendication précédente, la ressource de traitement (140) étant configurée pour utiliser la détection de grands cercles pour identifier la limite.

8. Système selon n'importe quelle revendication précédente, la ressource de traitement (140) étant configurée pour utiliser la détection de continuité pour identifier la limite.
